# EUROPEAN PATENT SPECIFICATION

(11) **EP 2 678 015 B1**
(45) Date of publication and mention of the grant of the patent: **26.11.2014**
(21) Application number: 12700487.7
(22) Date of filing: 19.01.2012
(51) Int. Cl.: A61K 31/422, A61K 45/06, A61P 31/04

(54) **USE OF NIFURATEL TO TREAT INFECTIONS CAUSED BY CLOSTRIDIUM SPECIES**
VERWENDUNG VON NIFURATEL ZUR BEHANDLUNG VON INFEKTIONEN, DIE VON EINER CHLOSTRIDIUM-SPEZIES VERURSACHT WERDEN
UTILISATION DE NIFURATEL POUR TRAITER DES INFECTIONS PROVOQUÉES PAR DES ESPÈCES DE CLOSTRIDIUM

(30) Priority: 22.02.2011 EP 11155283
(43) Date of publication of application: 01.01.2014
(73) Proprietor: Polichem SA, 1526 Luxembourg (LU)
(72) Inventor: BULGHERONI, Anna, I-21100 Varese (IT); MAILLAND, Federico, CH-6900 Lugano (CH); IOB, Giuliana, CH-6953 Lugaggia (CH)
(74) Representative: Pistolesi, Roberto
(86) International application number: PCT/EP2012/050759
(87) International publication number: WO 2012/113594

(56) References cited:
- US-A1- 2003 166 645
- ANANTHAKRISHNAN ASHWIN N: "Clostridium difficile infection: epidemiology, risk factors and management.", NATURE REVIEWS. GASTROENTEROLOGY & HEPATOLOGY JAN 2011 LNKD- PUBMED:21119612, vol. 8, no. 1, January 2011 (2011-01), pages 17-26, XP009148948, ISSN: 1759-5053
- SHEN E P ET AL: "Current treatment options for severe Clostridium difficile-associated disease", GASTROENTEROLOGY AND HEPATOLOGY 200802 US, vol. 4, no. 2, February 2008 (2008-02), pages 134-139, XP009148951, ISSN: 1554-7914

## Description

The present invention relates to the use of nifuratel, or a physiologically acceptable salt thereof, to treat infections caused by *Clostridium* species. The invention is further directed to the use of nifuratel to treat *Clostridium difficile* infection (CDI) and in particular *Clostridium difficile* associated diarrhoea (CDAD).

### BACKGROUND OF THE INVENTION

*Clostridium difficile,* a Gram-positive, spore forming, anaerobic bacillus, is a leading cause of antibiotic-associated diarrhoea in hospitals and long-term care facilities. *Clostridium difficile* infection (CDI)can arise when patients receive broad spectrum antibiotics (clindamycin being considered as the main responsible) that alter the ecological balance of the normal intestinal flora, allowing C. *difficile* proliferation and toxin production. Symptoms of CDI may vary from mild diarrhoea to life-threatening pseudomembranous colitis, fulminant colitis, toxic megacolon and death. These symptoms are mainly caused by the cytotoxic effects of toxin A and toxin B produced by C. *difficile.* Diagnosis of CDAD is made upon both clinical signs and symptoms and upon detection of toxin A and B in the stool using an enzyme-linked immunosorbent assay (Parkes G.C. et al. Lancet Infect Dis 2009, 9:237-44; Cohen S.H. et al. Infect control Hosp Epidemiol 2010, 31 (5):431-455).

Little is known about the extent of *Clostridium difficile* infection in Europe. Since 2003, increasing rates of C. *difficile* infections have been reported in Canada and USA, with a larger than previously reported proportion of severe and recurrent cases occurring in those countries.

The raised incidence and virulence of such infection have been partially explained by the indiscriminate use of antibiotics belonging to fluoroquinolone class, which caused the selection and spread of fluoroquinolone-resistant strains belonging to the PCR ribotype 027. Ribotype 027 was first reported in England in 2005, subsequently epidemics of this ribotype have been reported in many other European hospitals (Bauer MP et al. Lancet 2011, 377:63-73).

In the art, treatment of CDI is mainly limited to oral metronidazole or vancomycin both given for 7-14 days. Both treatments are highly effective against C. *difficile* and are equivalent in terms of overall response, although vancomycin therapy has been associated with a shorter mean duration of symptoms. Symptomatic recurrences are common following treatment with metronidazole or vancomycin. Many health authorities now recommend metronidazole as the first-line treatment agent because it is considerably less expensive than vancomycin, and in order to reduce the selective pressure for vancomycin- resistant enterococci (VRE)(Cohen S.H. et al. Infect control Hosp Epidemiol 2010, 31:431-455; Freeman J et al. JAC 2005, 56:717-725).

The severity of the consequences related to the current therapeutic options asks for an adequate treatment of CDI and CDAD. A few molecules are currently under development for the treatment of CDI, but they are in a very early stage. (Johnson A. P. Expert Opin Ther Patents 2010, 20:1389-1399). Thus, there is still an unsatisfied medical need in terms of efficacy and prevention of relapses.

Nifuratel is a nitrofurane derivative effective in the treatment of vaginal infections caused by protozoa (*Trichomonas vaginalis*) and by bacteria, such as *Gardnerella vaginalis* (Mendling W. et al. Arzneim Forsch Drug Res 2002, 52(1):8-13) or *Atopobium vaginae* (WO2010/121980).

It has now been surprisingly found that nifuratel, besides being active on vaginal pathogens, is also active against different *Clostridium* species.

### DESCRIPTION OF THE INVENTION

The object of the present invention is represented by nifuratel, or a physiologically acceptable salt thereof, for use in the treatment of any infection caused by *Clostridium* species, such as *Clostridium difficile, Clostridium butyricum* and *Clostridium beijerinckii.* More particularly, it is represented by the use of nifuratel, or a physiologically acceptable salt thereof, for treating *Clostridium difficile* infection (CDI) and, in particular, *Clostridium difficile* associated diarrhoea (CDAD).

Solid, semi-solid or liquid preparations of nifuratel or of a physiologically acceptable salt thereof, in the form of oral tablets, film-coated tablets, capsules, dragées or syrup, with a content in nifuratel from 10 to 1000 mg per single dose, more preferably from 50 to 800 mg per single dose, most preferably from 100 to 600 mg per single dose, are suitable to treat infections by *Clostridium* spp.; such preparations may be administered to infected patients according to conventional techniques; according to a preferred embodiment, they are administered on a regular basis, preferably three times daily. Pharmaceutical compositions may be prepared according to conventional techniques, may contain pharmaceutically acceptable excipients, adjuvants and/or carriers, and may also contain, in combination, one or more active principles with complementary or, in any case, useful activity.

The active agents which may be used in combination with nifuratel according to the present invention include, but are not limited to, antibiotics, antidiarrheal agents and probiotics; such active ingredients may be administered together with nifuratel (i.e. they may be for instance contained in the same composition as nifuratel) or they may be administered separately from or in temporal proximity with nifuratel.

Examples of antibiotics include metronidazole, vancomycin, bacitracine, rifaximine, aminoglycosides such as neomycin, gentamycin, amikacin, kanamycin and salts thereof.

Examples of the antidiarrheal agents include: bismuth subsalycilate, aluminium silicate, kaolin, activated charcoal, loperamide, attapulgite, zinc. Examples of probiotics include species of the genus *Lactobacillus, Bacillus clausii* and *Saccharomyces bouillardii.* Examples of the compositions prepared according to the present invention include: tablets, film-coated tablets, capsules, dragées or syrup suitable for oral administration.

The pharmaceutical compositions and the uses of the present invention will now be more fully described by the following examples. It should, however, be noted that such examples are given by way of illustration and not of limitation.

### EXAMPLE 1

An *in vitro* study was performed to investigate the susceptibility of 3 strains of *Clostridium difficile* (1 reference strain, ATCC 17858 and 2 clinical isolates), 1 reference strain of C. *butyricum* (ATCC 17791) and 2 reference strains of C. *beijerinckii* (ATCC 8260 and ATCC 17795) to nifuratel, compared to metronidazole and vancomycin. MICs were determined by the broth dilution method in *Brucella* broth (BB) supplemented with Hemin (5µg/mL), vitamin K1 (1µg/mL), laked horse blood (5%) and Oxidase (1:25 v/v), according to CLSI M11-A7. Nifuratel, vancomycin, metronidazole (previously dissolved in dimethyl sulfoxide) were added to the medium. The ranges of the concentrations tested were 0.125-128 µg/ml for all compounds.

The *in vitro* activity of nifuratel against the tested strains of *Clostridium difficile* (MIC range: ≤ 0.125 - 0.25 µg/ml) was comparable or better than metronidazole (MIC range 0.06 - 0.25 µg/ml) and vancomycin (MIC range: 0.25 - 0.5 µg/ml).

A quite good activity was observed also with *C. butyricum* and *C. beijerinckii.*

**Table 1: Comparison of MICs for different strains of Clostridium spp.**

| **Strain** | **Nifuratel µg/ml** | **Metronidazole µg/ml** | **Vancomycin µg/ml** |
|---|---|---|---|
| *C. difficile* ATCC 17858 | 0.25 | 0.25 | 0.5 |
| *C. difficile* | 0.25 | 0.06 | 0.25 |
| *C. difficile* | ≤0.125 | 0.125 | 0.25 |
| *C. butyricum* ATCC 17791 | 1 | 1 | 1 |
| *C. beijerinckii* ATCC 8260 | 0.5 | 0.125 | 0.5 |
| *C. beijerinckii* ATCC 17795 | 4 | 0.5 | 0.5 |

### EXAMPLE 2

Oral tablets are produced with the following quali-quantitative formula:

| Ingredients | Quantity (mg /tablet) |
|---|---|
| 1. Nifuratel | 200.00 |
| 2. Maize starch | 65.00 |
| 3. Talc | 30.00 |
| 4. Polyethylene glycol 6000 | 14.00 |
| 5. Magnesium stearate | 2.00 |

The process consists on the preparation (protecting from light) of binder solution with water and polyethylene glycol heat at 45°C under continuous stirring; then granulate in a Glatt fluid bed basket by blending nifuratel and starch until the mass is homogeneous, before spraying with the binder solution, then dry at inlet air temperature of 60°C; then add talc and magnesium stearate. Tabletting is done in a rotary tabletting machine with an appropriate punch.

The obtained tablet has a smooth surface with a yellow colour.

### EXAMPLE 3

A syrup having the following composition wt./wt.% is prepared:

| Ingredients | Quantity (g /100 ml syrup) |
|---|---|
| 1. Nifuratel | 4.0 |
| 2. Polysorbate 80 | 0.04 |
| 3. Sorbitol 70% | 20.0 |
| 4. Glycerol | 10.0 |
| 5. Sucrose | 30.0 |
| 6. Citric acid, monohydrate | 0.1 |
| 7. Methyl-parahydroxybenzoate | 0.07 |
| 8. Propyl-parahydroxybenzoate | 0.03 |
| 9. Sodium chloride | 0.04 |
| 10.Carboxymethyl cellulose | 0.6 |
| 11 Silicon dioxide | 2.0 |
| 12.Deionised water q.s. | ml 100 |

### Preparation

The formulation is prepared (protecting from light) as follows:
1) a gel is prepared with deionised water and carboxymethyl cellulose (3.75% in water). The gel is left to swallow overnight.
2) a solution of water, sucrose (50%) and sodium chloride (0.5%) is prepared apart.
3) a mixture of nifuratel (0.4%) and Polysorbate 80 (1%) is prepared in water. The mixture is stirred until it becomes homogeneous.
4) In a closed vessel with a stirrer are added deionised water, sorbitol and glycerol, the solution 2) and sucrose. The mixture is maintained under continuous stirring. Then add methyl-parahydroxybenzoate, propyl-parahydroxybenzoate and silicon dioxide. Heat at 100 °C under stirring for 30 minutes. Cooling at 80°C citric acid is added. Then cooling at 40°C, add the gel 1) and the preparation 3) under continuous stirring.

The resulting syrup is a homogeneous suspension.

### EXAMPLE 4

Oral gastro-resistant tablets, whose cores are produced as for example 2, are film-coated with a spray-suspension with the following quali-quantitative composition:

| Ingredients | |
|---|---|
| 1. Methacrylic Acid Copolymer | 10.0% |
| 2. Triethyl Citrate | 5.5% |
| 3. Talc | 4.5% |
| 4. Titanium Dioxide | 0.2% |
| 5. 1N NaOH | 5.5% |
| 6. Water | 74.3% |

Procedure: add methacrylic acid copolymer slowly into 2/3 of the water and stir until the powder is completely wetted. Add, on sequence, the 1 N NaOH solution and triethylcitrate into the polymer suspension and stir after each addition for about 60 minutes. In a separate vessel, homogenize talc and titanium dioxide in the remaining 1/3 of water for about 10 minutes and pour the obtained suspension into the polymer dispersion while stirring. Maintain the obtained spray-suspension on agitation. Transfer the tablets, prepared as for example 2, into a suitable equipped pan. Set temperature at about 40°C and start spraying with the previously obtained suspension, under moderate rotation speed. At the end of the coating process, start drying stirring and cool down at 25-30°C.

### EXAMPLE 5

Oral gastro-resistant tablets, whose cores are produced as for example 2, are film-coated with a spray-suspension with the following quali-quantitative composition:

| Ingredients | |
|---|---|
| 1. Cellulose Acetate Pthalate | 12.0% |
| 2. Diethyl Pthalate | 4.0% |
| 3. Peg-6000 | 1.5% |
| 4. Water | 82.5% |

Procedure: add cellulose acetate pthalate into 1/3 of the water and stir until to obtain an homogeneous dispersion. Add diethyl pthalate and mix for about 30 minutes. In a separate vessel solubilize Peg-6000 in the remaining 2/3 of water and add the solution obtained to the cellulose pthalate dispersion. Mix for about 10 minutes. Proceed applying the coating to the tablets as for example 4 setting temperature at 70-80°C.

### EXAMPLE 6

Oral gastro-resistant capsules, filled with the granules produced as for example 2 without the tabletting process, are film-coated with the spray-suspension described in example 4 or in example 5.

### EXAMPLE 7

Oral gastro-resistant dragèes, whose cores are produced as for example 2, are film-coated with the spray-suspension described in example 4 or in example 5 and finally sugar coated with the following composition, using a proper coating pan:

| | |
|---|---|
| 1.Sucrose | 54.00% |
| 2.Corn Starch | 3.50% |
| 3.Titanium Dioxide | 0.30% |
| 4.Magnesium carbonate | 11.00% |
| 5.Gelatine | 0.75% |
| 6.Arabic Gum | 0.75% |
| 7.Wax | 0.10% |
| 8.Water | 29.60% |

## Claims

1. Nifuratel or a physiologically acceptable salt thereof, for use in the treatment of any infection caused by one or more species of the genus *Clostridium.*

2. Nifuratel for use according to claim 1, **characterized in that** said species is selected from *Clostridium difficile, Clostridium butyricum* and *Clostridium beijerinckii.*

3. Nifuratel for use according to claim 2, **characterized in that** said species is *Clostridium difficile.*

4. Nifuratel for use according to any of the preceding claims, **characterized in that** said infection is selected from intestinal tox-infections.

5. Nifuratel for use according to claim 4, **characterized in that** said intestinal tox-infection is *Clostridium difficile* associated diarrhoea.

6. Nifuratel for use according to any of the preceding claims, **characterized in that** it is administered in the form of a solid, semi solid or liquid pharmaceutical formulation.

7. Nifuratel for use according to claim 6, **characterized in that** said pharmaceutical formulation is selected from: tablets, film-coated tablets, capsules, dragées or syrup suitable for oral administration.

8. Nifuratel for use according to claims 6 or 7, **characterized in that** said formulation has a content in nifuratel or a salt thereof, from 10 to 1000 mg per single dose.

9. Nifuratel for use according to claims 6 or 7, **characterized in that** said formulation has a content in nifuratel or a salt thereof, from 50 to 800 mg per single dose.

10. Nifuratel for use according to claims 6 or 7, **characterized in that** said formulation has a content in nifuratel or a salt thereof, from 100 to 600 mg per single dose.

11. Nifuratel for use according to any of the preceding claims, **characterized in that** it is administered in combination or in temporal proximity with at least one active principle selected from antibiotics, probiotics, anti-diarrhoea agents.

12. Nifuratel for use according to claim 11, **characterized in that** said at least one antibiotic is selected from: metronidazole, vancomycin, bacitracine, rifaximine, aminoglycosides such as neomycin, gentamycin, amikacin, kanamycin and salts thereof.

13. Nifuratel for use according to claim 11, **characterized in that** said at least one antidiarrheal agent is selected from: bismuth subsalycilate, aluminium silicate, kaolin, activated charcoal, loperamide, attapulgite and zinc.

14. Nifuratel for use according to claim 11, **characterized in that** said at least one probiotic is selected from species of the genus *Lactobacillus, Bacillus clausii* and *Saccharomyces bouillardii.*

## Patentansprüche

1. Nifuratel oder ein physiologisch akzeptables Salz davon, zur Verwendung bei der Behandlung von irgendeiner Infektion, die durch eine oder mehrere Art(en) der Gattung *Clostridium* verursacht wurde.

2. Nifuratel zur Verwendung gemäß Anspruch 1, **dadurch gekennzeichnet, dass** diese Art(en) ausgewählt ist (sind) aus *Clostridium difficile, Clostridium butyricum* und *Clostridium beijerinckii.*

3. Nifuratel zur Verwendung gemäß Anspruch 2, **dadurch gekennzeichnet, dass** diese Art *Clostridium difficile* ist.

4. Nifuratel zur Verwendung gemäß irgendeinem der vorhergehenden Ansprüche, **dadurch gekennzeichnet, dass** diese Infektion ausgewählt ist aus intestinalen Toxi-Infektionen.

5. Nifuratel zur Verwendung gemäß Anspruch 4, **dadurch gekennzeichnet, dass** diese intestinale Toxi-Infektion eine *Clostridium difficile*-assoziierte Diarrhö ist.

6. Nifuratel zur Verwendung gemäß irgendeinem der vorhergehenden Ansprüche, **dadurch gekennzeichnet, dass** es in Form einer festen, halbfesten oder flüssigen pharmazeutischen Formulierung verabreicht wird.

7. Nifuratel zur Verwendung gemäß Anspruch 6, **dadurch gekennzeichnet, dass** diese pharmazeutische Formulierung ausgewählt ist aus: Tabletten, Filmtabletten, Kapseln, Dragees oder Sirup geeignet zur oralen Verabreichung.

8. Nifuratel zur Verwendung gemäß den Ansprüchen 6 oder 7, **dadurch gekennzeichnet, dass** diese Formulierung einen Gehalt an Nifuratel oder eines Salzes davon von 10 bis 1000 mg pro Einzeldosis hat.

9. Nifuratel zur Verwendung gemäß den Ansprüchen 6 oder 7, **dadurch gekennzeichnet, dass** diese Formulierung einen Gehalt an Nifuratel oder eines Salzes davon von 50 bis 800 mg pro Einzeldosis hat.

10. Nifuratel zur Verwendung gemäß den Ansprüchen 6 oder 7, **dadurch gekennzeichnet, dass** diese Formulierung einen Gehalt an Nifuratel oder eines Salzes davon von 100 bis 600 mg pro Einzeldosis hat.

11. Nifuratel zur Verwendung gemäß irgendeinem der vorhergehenden Ansprüche, **dadurch gekennzeichnet, dass** es in Kombination oder in zeitlicher Nähe mit mindestens einem Wirkprinzip ausgewählt aus Antibiotika, Probiotika, Anti-Diarrhö-Agenzien, verabreicht wird.

12. Nifuratel zur Verwendung gemäß Anspruch 11, **dadurch gekennzeichnet, dass** dieses mindestens eine Antibiotikum ausgewählt ist aus: Metronidazol, Vancomycin, Bacitracin, Rifaximin, Aminoglykoside wie Neomycin, Gentamicin, Amikacin, Kanamycin und Salze davon.

13. Nifuratel zur Verwendung gemäß Anspruch 11, **dadurch gekennzeichnet, dass** dieses mindestens eine Anti-Diarrhö-Agens ausgewählt ist aus: Bismutsubsalicylat, Aluminiumsilikat, Kaolin, Aktivkohle, Loperamid, Attapulgit und Zink.

14. Nifuratel zur Verwendung gemäß Anspruch 11, **dadurch gekennzeichnet, dass** dieses mindestens eine Probiotikum ausgewählt ist aus den Arten der Gattung *Lactobacillus, Bacillus clausii* und *Saccharomyces boulardii.*

## Revendications

1. Nifuratel ou sel physiologiquement acceptable de celui-ci, pour une utilisation dans le traitement d'une quelconque infection causée par une ou plusieurs espèces du genre *Clostridium.*

2. Nifuratel pour une utilisation selon la revendication 1, **caractérisé en ce que** ladite espèce est choisie parmi *Clostridium difficile, Clostridium butyricum* et *Clostridium beijerinckii.*

3. Nifuratel pour une utilisation selon la revendication 2, **caractérisé en ce que** ladite espèce est *Clostridium difficile.*

4. Nifuratel pour une utilisation selon l'une quelconque des revendications précédentes, **caractérisé en ce que** ladite infection est choisie parmi les toxi-infections intestinales.

5. Nifuratel pour une utilisation selon la revendication 4, **caractérisé en ce que** ladite toxi-infection intestinale est une diarrhée associée à *Clostridium difficile.*

6. Nifuratel pour une utilisation selon l'une quelconque des revendications précédentes, **caractérisé en ce qu'**il est administré sous la forme d'une formulation pharmaceutique solide, semi-solide ou liquide.

7. Nifuratel pour une utilisation selon la revendication 6, **caractérisé en ce que** ladite formulation pharmaceutique est choisie parmi les comprimés, les comprimés pelliculés, les gélules, les dragées ou un sirop approprié pour une administration par voie orale.

8. Nifuratel pour une utilisation selon la revendication 6 ou 7, **caractérisé en ce que** ladite formulation a une teneur en nifuratel ou un sel de celui-ci de 10 à 1000 mg par dose unique.

9. Nifuratel pour une utilisation selon la revendication 6 ou 7, **caractérisé en ce que** ladite formulation a une teneur en nifuratel ou un sel de celui-ci de 50 à 800 mg par dose unique.

10. Nifuratel pour une utilisation selon la revendication 6 ou 7, **caractérisé en ce que** ladite formulation a une teneur en nifuratel ou un sel de celui-ci de 100 à 600 mg par dose unique.

11. Nifuratel pour une utilisation selon l'une quelconque des revendications précédentes, **caractérisé en ce qu'**il est administré en association ou dans un temps rapproché avec au moins un principe actif choisi parmi les antibiotiques, les probiotiques et les agents anti-diarrhée.

12. Nifuratel pour une utilisation selon la revendication 11, **caractérisé en ce que** ledit au moins un antibiotique est choisi parmi le métronidazole, la vancomycine, la bacitracine, la rifaximine, les aminoglycosides tels que la néomycine, la gentamycine, l'amikacine, la kanamycine et leurs sels.

13. Nifuratel pour une utilisation selon la revendication 11, **caractérisé en ce que** ledit au moins un agent anti-diarrhée est choisi parmi le subsalycilate de bismuth, le silicate d'aluminium, le kaolin, le charbon actif, le lopéramide, l'attapulgite et le zinc.

14. Nifuratel pour une utilisation selon la revendication 11, **caractérisé en ce que** ledit au moins un probiotique est choisi parmi les espèces du genre *Lactobacillus, Bacillus clausii* et *Saccharomyces bouillardii.*
